# EUROPEAN PATENT SPECIFICATION

(11) **EP 2 474 536 B1**
(45) Date of publication and mention of the grant of the patent: **25.06.2014**
(21) Application number: 10811488.5
(22) Date of filing: 23.08.2010
(51) Int. Cl.: C07D 239/52, C07C 201/08, C07C 201/12, C07C 205/26, C07C 205/34, C07C 205/58, C07C 205/19

(54) **PROCESS FOR PREPARATION OF PYRIMIDINYLACETONITRILE DERIVATIVES AND INTERMEDIATES FOR SYNTHESIS THEREOF**
VERFAHREN ZUR HERSTELLUNG VON PYRIMIDINYLACETONITRILDERIVATEN UND ZWISCHENPRODUKTE FÜR DEREN SYNTHESE
PROCÉDÉ DE PRÉPARATION DE DÉRIVÉS DE PYRIMIDINYL-ACÉTONITRILE ET D'INTERMÉDIAIRES POUR LEUR SYNTHÈSE

(30) Priority: 31.08.2009 JP 2009200158
(43) Date of publication of application: 11.07.2012
(73) Proprietor: Ihara Chemical Industry Co., Ltd., Tokyo 110-0008 (JP)
(72) Inventor: KAWAZOE, Kentaro, Fuji-shi Shizuoka 421-3306 (JP)
(74) Representative: Vossius & Partner
(86) International application number: PCT/JP2010/005171
(87) International publication number: WO 2011/024429

(56) References cited:
- EP-A1- 1 101 760
- WO-A1-03/062210
- WO-A1-2008/145963
- WO-A2-2006/099379
- US-A- 3 312 700
- BIANCO A. ET AL: 'Improved procedure for the reduction of esters to alcohols by sodium borohydride' SYNTHETIC COMMUNICATIONS vol. 18, no. 15, 1988, pages 1765 - 1771, XP008068843

## Description

### TECHNICAL FIELD

The present invention relates to a process for producing a pyrimidinylacetonitrile derivative and an intermediate for synthesis of the derivative.

### BACKGROUND ART

As to (5-chloro-3-methoxymethyl-2-nitrophenyl)(4,6-dimethoxypyrimidin-2-yl)acetonitrile (which is a pyrimidinylacetonitrile derivative), it is known that this compound can be derived to (2-amino)-3-methoxymethylphenyl)(4,6-dimethoxypyrimidin-2-yl)methanol (which is an intermediate for synthesis of herbicide) via (5-chloro-3-methoxymethyl-2-nitrophenyl)(4,6-dimethoxypyrimidin-2-yl)ketone obtained by the oxidation of the above compound (reference is made to Patent Literature 1).

For obtaining the (5-chloro-3-methoxymethyl-2-nitrophenyl)(4,6-dimethoxypyrimidin-2-yl)acetonitrile, there is known a method of reacting a 4-halogeno-2-alkoxymethylnitrobenzene with an acetonitrile substituted with substituted phenoxy group, in the presence of a base to obtain a 5-halogeno-3-alkoxymethyl-2-nitrophenylacetonitrile and then reacting it with 4,6-dimethoxy-2-methylsulfonylpyrimidine in the presence of a base (reference is made to Patent Literature 1). In this method, however, the reaction of 4-chloro-2-methoxymethylnitrobenzene with 4-chlorophenoxyacetonitrile is conducted at a low temperature (-50°C to -20°C) and the yield of desired product is 23%, in Example 2 of the Patent Literature 1. Thus, it has been desired to develop a method suitable for industrial application.

### PRIOR ART LITERATURE

### Patent Literature

Patent Literature 1: JP-A-2003-212861

### SUMMARY OF THE INVENTION

### Task to Be Achieved by the Invention

The present invention has been made in order to solve the above-mentioned problem of the prior art and provide a process for producing a pyrimidinylacetonitrile derivative represented by (5-chloro-3-methoxymethyl-2-nitrophenyl)(4,6-dimethoxypyrimidin-2-yl)acetonitrile easily and efficiently from industrially available raw materials.

### Means for Achieving the Task

In view of the above situation, the present inventor made a study on a process for producing a pyrimidinylacetonitrile derivative represented by (5-chloro-3-methoxymethyl-2-nitrophenyl)(4,6-dimethoxypyrimidin-2-yl)acetonitrile. As a result, it was unexpectedly found that the above task could be achieved by reacting a 2,4-dihalogenonitrobenzene compound with 4,6-dimethoxy-2-cyanomethylpyrimidine and that the 2,4-dihalogenonitrobenzene compound could be derived from a 3,5-dihalogeno-2-nitrobenzoic acid compound or an alkyl 3,5-dihalogenobenzoate compound, both of good industrial availability. The finding has led to the completion of the present invention. Incidentally, the 2,4-dihalogenonitrobenzene compound and the 2,4-dihalogeno-6-hydroxymethylnitrobenzene compound (which is a precursor of the former compound) are novel compounds.

The present invention has achieved the above-mentioned task by providing the following inventions [1] to [7] .
[1] A process for producing a pyrimidinylacetonitrile derivative represented by the general formula (3) 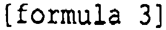 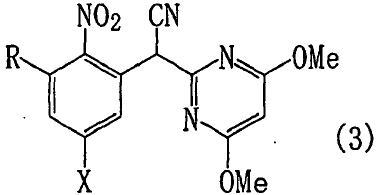 (wherein X is a halogen atom, R is an alkoxymethyl group, and Me is a methyl group), characterized by reacting, in the presence of a base, a 2,4-dihalogenonitrobenzene compound represented by the general formula (1) 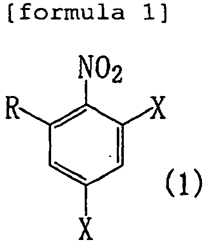 (wherein X and R are as defined above) with 4,6-dimehtoxy-2-cyanomethylpyrimidine represented by a formula (2) 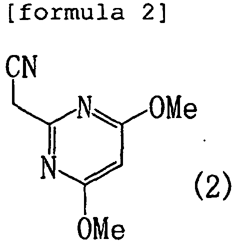 (wherein Me is as defined above).
[2] A process for producing a pyrimidinylacetonitrile derivative set forth in [1], wherein the 2,4-dihalogenonitrobenzene compound represented by the general formula (1) 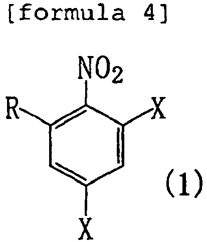 (wherein X is a halogen atom, and R is an alkoxymethyl group) is produced by subjecting a 3,5-dihalogeno-2-nitrobenzoic acid compound represented by the general formula (4) 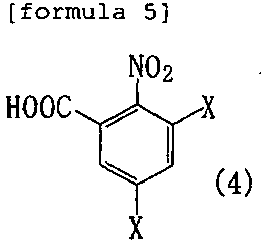 (wherein X is as defined above) to alkylation in the presence of a base (method A), or, subjecting an alkyl 3,5-dihaogenobenzoate compound represented by the general formula (5) 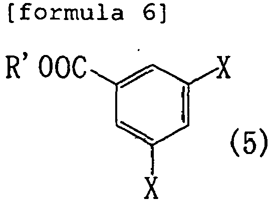 (wherein R' is an alkyl group, and X is as defined above) to nitration (method B), to obtain an alkyl 3,5-dihalogeno-2-nitrobenzoate compound represented by the general formula (6) 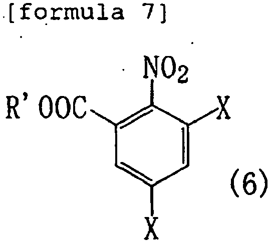 (wherein X and R' are as defined above), then reducing the alkyl 3,5-dihalogeno-2-nitrobenzoate compound to obtain a 2,4-dihalogeno-6-hydroxymethylnitrobenzene compound represented by the general formula (7) 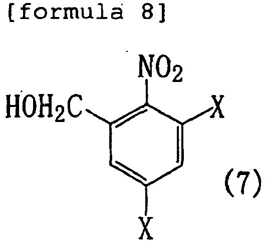 (wherein X is as defined above), and subjecting the 2,4-dihalogeno-6-hydroxymethylnitrobenzene compound to alkylation in the presence of a base.
[3] A process for producing a pyrimidinylacetonitrile derivative set forth in [2], wherein the alkyl 3,5-dihalogeno-2-nitrobenzoate compound represented by the general formula (6) 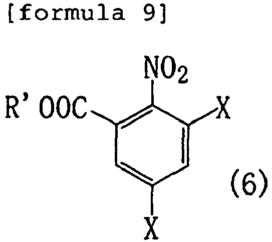 (wherein R' is an alkyl group, and X is a halogen atom) is produced by subjecting a 3,5-dihalogeno-2-nitrobenzoic acid compound represented by the general formula (4) 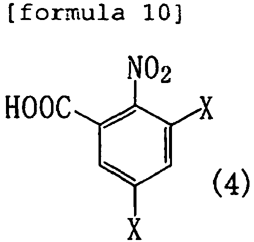 (wherein X is as defined above) to alkylation in the presence of a base (method A).
[4] A process for producing a pyrimidinylacetonitrile derivative set forth in [2], wherein the alkyl 3,5-dihalogeno-2-nitrobenzoate compound represented by the general formula (6) 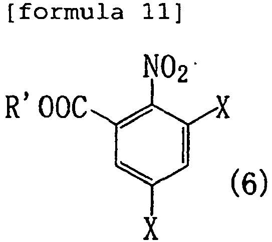 (wherein R' is an alkyl group, and X is a halogen atom) is produced by subjecting an alkyl 3,5-dihaogenobenzoate compound represented by the general formula (5) 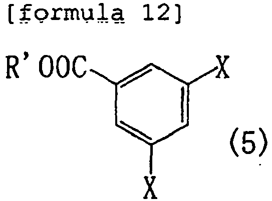 (wherein R' and X are as defined above) to nitration (method B) .
[5] A 2,4-dihalogenonitrobenzene compound represented by the general formula (1) 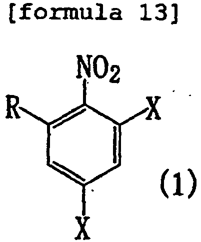 (wherein X is a halogen atom, and R is an alkoxymethyl group).
[6] A 2,4-dihalogeno-6-hydroxymethylnitrobenzene compound represented by the general formula (7) 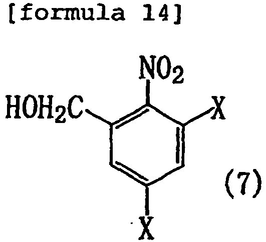 (wherein X is a halogen atom).
[7] A process for producing a pyrimidinylacetonitrile derivative set forth in [1], wherein the 2,4-dihalogenonitrobenzene compound represented by the general formula (1) 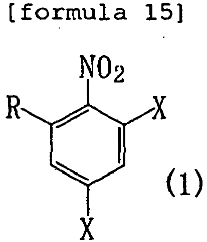 (wherein X is a halogen atom, and R is an alkoxymethyl group) is produced by reducing an alkyl 3,5-dihalogeno-2-nitrobenzoate compound represented by the general formula (6) 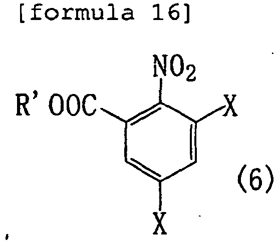 (wherein R' is an alkyl group, and X is a halogen atom) to obtain a 2,4-dihalogeno-6-hydroxymethylnitrobenzene compound represented by the general formula (7) 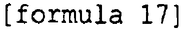 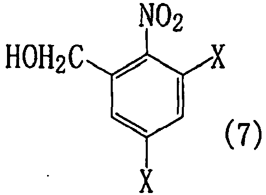 (wherein X is as defined above), and subjecting the 2,4-dihalogeno-6-hydroxymethylnitrobenzene compound to alkylation in the presence of a base.

### Effect of the Invention

The process of the present invention provides a novel process for industrial production of pyrimidinylacetonitrile derivative. According to the present process, an intended pyrimidinylacetonitrile derivative can be produced at a high selectivity, efficiently, in an easy operation, and under mild conditions, without using any special reactor, by using, as raw materials, a 3,5-dihalogeno-2-nitrobenzoic acid or an alkyl 3,5-dihalogenobenzoate compound, both of good availability. Further, the 3,5-dihalogeno-2-nitrobenzoic acid compound and the alkyl 3,5-dihalogenobenzoate compound can be produced from a 3,5-dihalogenobenzoic acid compound which is a symmetric substance of simple structure; therefore, according to the present process, it has become possible to produce, from raw materials of good availability, a pyrimid-inylacetonitrile derivative having phenyl group of an asymmetric structure, e.g. (5-chloro-3-methoxymethyl-2-nitrophenyl)(4,6-dimethoxypyrimidin-2-yl)acetonitrile, selectively and at a high yield.

### BEST MODE FOR CARRYING OUT THE INVENTION

The present invention is described in detail below.

Description is made first on the 2,4-dihalogenonitrobenzene compound represented by the general formula (1).

In the general formula (1), the substituent X is any of halogen atoms consisting of fluorine, chlorine, bromine and iodine.

In the general formula (1), the substituent R is a methyl group substituted with a straight chain or branched chain alkoxy group of 1 to 6 carbon atoms [hereinafter, the carbon numbers of the substituent may be abbreviated as "C1 to C6" (this case)], that is, a (C1 to C6 alkoxy)-methyl group. As specific examples thereof, there can be mentioned methoxymethyl group, ethoxymethyl group, propoxymethyl group, butoxymethyl group, pentoxymethyl group and hexoxymethyl group.

Therefore, as specific examples of the 2,4-dihalogenonitrobenzene compound represented by the general formula (1), there can be mentioned 2,4-dichloro-6-methoxymethylnitrobenzene, 2,4-dibromo-6-methoxymethylnitrobenzene, 2,4-difluoro-6-methoxymethylnitrobenzene, 2,4-diiodo-6-methoxymethylnitrobenzene, 2,4-dichloro-6-ethoxymethylnitrobenzene, 2,4-dibromo-6-ethoxymethylnitrobenzene, 2,4-difluoro-6-ethoxymethylnitrobenzene, 2,4-diiodo-6-ethoxymethylnitrobenzene, 2,4-dichloro-6-propoxymethylnitrobenzene, 2,4-dibromo-6-propoxymethylnitrobenzene, 2,4-difluoro-6-propoxymethylnitrobenzene, 2,4-diiodo-6-propoxymethylnitrobenzene, 2,4-dichloro-6-isopropoxymethylnitrobenzene, 2,4-dibromo-6-isopropoxymethylnitrobenzene, 2,4-difluoro-6-isopropoxymethylnitrobenzene, 2,4-diiodo-6-isopropoxymethylnitrobenzene, 2,4-dichloro-6-butoxymethylnitrobenzene, 2,4-dibromo-6-pentoxymethylnitrobenzene, and 2,4-difluoro-6-hexoxymethylnitrobenzene. Incidentally, the 2,4-dihalogenonitrobenzene compound represented by the general formula (1) is a novel compound.

The process of the present invention is a process for producing a pyrimidinylacetonitrile derivative represented by the general formula (3), characterized by reacting a 2,4-dihalogenonitrobenzene compound represented by the general formula (1) with 4,6-dimethoxy-2-cyanomethylpyrimidine represented by a formula (2) in the presence of a base.

In the 4,6-dimethoxy-2-cyanomethylpyrimidine represented by the formula (2) and the pyrimidinylacetonitrile derivative represented by the general formula (3), the substituent Me is a methyl group and, in the pyrimidinylacetonitrile derivative represented by the general formula (3), the substituents X and R have the same meanings as the X and R in the 2,4-dihalogenonitrobenzene compound represented by the general formula (1).

Accordingly, as the pyrimidinylacetonitrile derivative represented by the general formula (3), obtained in the present reaction, there can be mentioned a compound having the same substituents X and R as in the 2,4-dihalogenonitrobenzene compound represented by the general formula (1).

In the present reaction, a pyrimidinylacetonitrile derivative represented by the general formula (3) can be obtained selectively by subjecting only the 2-position halogen of the 2,4-dihalogenonitrobenzene compound represented by the general formula (1), to a selective nucleophilic substitution reaction.

The molar ratio of the 2,4-dihalogenonitrobenzene compound represented by the general formula (1) and the 2-cyanomethyl-4,6-dimethoxypyrimidine represented by the formula (2), both used in the reaction, may be any molar ratio and the reaction proceeds satisfactorily. However, the proportion of the 2-cyanomethyl-4,6-dimethoxypyrimidine represented by the formula (2) is, for example, ordinarily 0.1 to 10.0 mols, preferably 0.5 to 2.0 mols, more preferably 0.8 to 1.2 mols relative to 1 mol of the 2,4-dihalogenonitrobenzene compound represented by the general formula (1).

The reaction proceeds in the presence of a base. As specific examples of the base used in the reaction, there can be mentioned inorganic bases such as alkali metal hydroxides (represented by sodium hydroxide, potassium hydroxide, lithium hydroxide, etc.), alkaline earth metal hydroxides (represented by barium hydroxide, calcium hydroxide, etc.), alkali metal carbonates (represented by potassium carbonate, sodium carbonate, etc.), alkali metal hydrogencarbonates (represented by potassium hydrogencarbonate, sodium hydrogencarbonate, etc.), and the like, or aqueous solutions of the inorganic bases; alkali metal hydrides such as sodium hydride and the like; and organic bases such as pyridine and tertiary amine compounds (e.g. N,N-diisopropylethylamine and triethylamine), and the like. These bases may be used singly or in mixed base of any proportions. Preferred from the standpoints of availability, easy handling, reactivity, etc. are inorganic bases such as sodium hydroxide, potassium hydroxide, potassium carbonate, sodium carbonate, potassium hydrogencarbonate, sodium hydrogencarbonate and the like, and more preferred are sodium hydroxide.

The molar ratio of the 2,4-dihalogenonitrobenzene compound represented by the general formula (1) and the base, both used in the reaction, may be any molar ratio and the reaction proceeds satisfactorily. However, the proportion of the base is, for example, ordinarily 0.2 to 10.0 mols, preferably 1.0 to 4.0 mols, more preferably 2.0 to 3.0 mols relative to 1 mol of the 2,4-dihalogenonitrobenzene compound represented by the general formula (1).

The reaction may be carried out in a solvent-free state. However, use of a solvent is preferred for smooth progress of the reaction. The solvent used in the reaction may be any solvent as long as it does not inhibit the reaction. As the solvent, there can be mentioned, for example, aprotic polar solvents such as N,N-dimethylformamide (DMF), N,N-dimethylacetamide (DMAC), acetonitrile, propylene carbonate and the like; aromatic hydrocarbons such as toluene, xylene, chlorobenzene and the like; halogen-containing solvents such as dichloromethane and the like; ethers such as phenyl ether, diethyl ether, tetrahydrofuran (THF), dioxane and the like; and aliphatic hydrocarbons such as pentane, n-hexane and the like. Preferred are aprotic polar solvents such as N,N-dimethylformamide (DMF), N,N-dimethylacetamide (DMAC), acetonitrile, propylene carbonate and the like. Particularly preferred is N,N-dimethylformamide (DMF).

The solvents may be used singly or in mixed solvent of any mixing proportions.

The amount of the solvent may be any amount as long as it allows for sufficient mixing of the reaction system. However, the amount is ordinarily 0 to 10 liters, preferably 0.05 to 10 liters, more preferably 0.2 to 2 liters relative to 1 mol of the 2,4-dihalogenonitrobenzene compound represented by the general formula (1).

The temperature of the reaction is, for example, 0°C to the refluxing temperature of the solvent used, preferably 0 to 100°C.

The time of the reaction is not restricted particularly, but is preferably 1 hour to 30 hours in view of, for example, the prevention of by-product formation.

The 2,4-dihalogenonitrobenzene compound represented by the general formula (1), used as a raw material of the present process can be easily derived from the 3,5-dihalogeno-2-nitrobenzoic acid compound represented by the general formula (4) which can be derived from an industrially available 3,5-dihalogenobenzoic acid compound of simple structure having symmetry, or from the alkyl 3,5-dihalogeno-2-nitrobenzoate compound represented by the general formula (5).

Specifically, the 2,4-dihalogenonitrobenzene compound represented by the general formula (1) can be obtained by subjecting a 3,5-dihalogeno-2-nitrobenzoic acid compound represented by the general formula (4) to alkylation in the presence of a base (method A) or subjecting an alkyl 3,5-dihaogenobenzoate compound represented by the general formula (5) to nitration (method B), to obtain an alkyl 3,5-dihalogeno-2-nitrobenzoate compound represented by the general formula (6), then reducing the alkyl 3,5-dihalogeno-2-nitrobenzoate compound to obtain a 2,4-dihalogeno-6-hydroxymethylnitrobenzene compound represented by the general formula (7), and subjecting the 2,4-dihalogeno-6-hydroxymethylnitrobenzene compound to alkylation.

Here, description is made on the 3,5-dihalogeno-2-nitrobenzoic acid compound represented by the general formula (4).

In the general formula (4), the substituent X is as defined above. Therefore, as specific examples of the 3,5-dihalogeno-2-nitrobenzoic acid compound represented by the general formula (4), there can be mentioned 3,5-dichloro-2-nitrobenzoic acid, 3,5-dibromo-2-nitrobenzoic acid, 3,5-difluoro-2-nitrobenzoic acid, and 3,5-diiodo-2-nitrobenzoic acid.

The 3,5-dihalogeno-2-nitrobenzoic acid compound represented by the general formula (4) is a known compound, or can be produced, for example, by a method of subjecting a corresponding 3,5-dihalogenobenzoic acid compound (a symmetric compound) to nitration, or a method of oxidizing the amino group of a corresponding 3,5-dihalogeno-2-aminobenzoic acid compound, or a method of subjecting a corresponding 3,5-dihalogeno-2-aminobenzoic acid (used as a raw material), to diazotization of amino group and then reacting the resulting compound with a nitrite.

Successively, description is made on the alkyl 3,5-dihalogenobenzoate compound represented by the general formula (5).

In the general formula (5), the substituent R' is a C1 to C6 alkyl group such as methyl group, ethyl group, n-propyl group, isopropyl group, n-butyl group, isobutyl group, n-pentyl group, n-hexyl group or the like; and the substituent X is as defined above.

Therefore, as specific examples of the alkyl 3,5-dihalogenobenzoate compound represented by the general formula (5), there can be mentioned methyl 3,5-dichlorobenzoate, methyl 3,5-dibromobenzoate, methyl 3,5-difluorobenzoate, methyl 3,5-diiodobenzoate, ethyl 3,5-dichlorobenzoate, ethyl 3,5-dibromobenzoate, ethyl 3,5-difluorobenzoate, ethyl 3,5-diiodobenzoate, n-propyl 3,5-dichlorobenzoate, n-butyl 3,5-dibromobenzoate, n-pentyl 3,5-difluorobenzoate, isopropyl 3,5-diiodobenzoate, and n-hexyl 3,5-dichlorobenzoate.

The alkyl 3,5-dihalogeno-benzoate compound represented by the general formula (5) is a known compound, or can be produced, for example, by a method of subjecting a corresponding 3,5-dihalogenobenzoic acid (raw material) to alkylation, or a method of converting a corresponding 3,5-dihalogenobenzoic acid (raw material) to an acid halide by a conventional means (e.g. a reaction with thionyl halide) and then subjecting the resulting compound to alkyl esterification.

Next, description is made on the process for producing an alkyl 3,5-dihalogeno-2-nitrobenzoate compound represented by the general formula (6).

The alkyl 3,5-dihalogeno-2-nitrobenzoate compound represented by the general formula (6) can be produced by subjecting a 3,5-dihalogeno-2-nitrobenzoic acid compound represented by the general formula (4) to alkylation with an alkylating agent in the presence of a base (method A), or by subjecting an alkyl 3,5-dihalogenobenzoate compound represented by the general formula (5) to nitration with a nitrating agent (method B).

### Method A

The alkylation of the 3,5-dihalogeno-2-nitrobenzoic acid compound represented by the general formula (4) is conducted using an alkylating agent represented by a methylating agent. The alkylating agent used in the alkylation may be any alkylating agent as long as it can give rise to the alkylation. The alkylating agent may be used singly or in mixed agent of any mixing proportions.

Therefore, as specific examples of the alkylating agent usable in the reaction, there can be mentioned alkylating agents capable of introducing a "C1 to C6 alkyl group", such as dialkyl sulfate (represented by dimethyl sulfate, diethyl sulfate, di-n-propyl sulfate or di-isopropyl sulfate), alkyl alkylsulfonate or alkyl arylsulfonate (represented by methyl methanesulfonate, methyl toluenesulfonate, ethyl methanesulfonate, ethyl toluenesulfonate, n-propyl methanesulfonate, n-propyl toluenesulfonate, isopropyl methanesulfonate, isopropyl toluenesulfonate, n-butyl methanesulfonate, n-pentyl methanesulfonate or n-hexyl methanesulfonate), alkyl halide (represented by methyl iodide, methyl bromide, methyl chloride, ethyl iodide, ethyl bromide, ethyl chloride, propyl iodide, propyl bromide, propyl chloride, isopropyl iodide, isopropyl bromide, isopropyl chloride, butyl bromide, pentyl bromide, hexyl bromide or the like), alcohol (represented by methanol, ethanol, n-propanol or isopropanol), and the like. As specific examples of the alkyl group introduced, there can be mentioned methyl group, ethyl group, n-propyl group, isopropyl group, n-butyl group, isobutyl group, n-pentyl group and n-hexyl group and the like. A dialkyl sulfate or an alkyl alkylsulfonate is used preferably from the standpoints of availability, easy handling, reactivity, etc., and a methylating agent such as dimethyl sulfate or methyl methanesulfonate is used more preferably, and dimethyl sulfate is used particularly preferably.

The molar ratio of the 3,5-dihalogeno-2-nitrobenzoic acid represented by the general formula (4) and the alkylating agent, both used in the reaction, may be any molar ratio and the reaction proceeds satisfactorily. However, the proportion of the alkylating agent is, for example, ordinarily 0.1 to 10.0 mols, preferably 0.33 to 3.0 mols, more preferably 1.0 to 2.0 mols, relative to 1 mol of the 3,5-dihalogeno-2-nitrobenzoic acid represented by the general formula (4).

The reaction proceeds in the presence of a base. As specific examples of the base used in the reaction, there can be mentioned inorganic bases such as alkali metal carbonates (represented by potassium carbonate and sodium carbonate), alkali metal hydrogencarbonates (represented by potassium hydrogencarbonate and sodium hydrogencarbonate), alkali metal hydroxides (represented by potassium hydroxide, sodium hydroxide and lithium hydroxide) and the like; and organic bases such as pyridine, tertiary amine compounds (e.g. N,N-diisopropylethylamine and triethylamine) and the like. These bases may be used singly or in mixed base of any proportions. Preferred from the standpoints of availability, easy handling, reactivity, etc. are inorganic bases, and more preferred are alkali metal carbonates, alkali metal hydroxides and alkali metal hydrogencarbonates. Specifically, preferred are potassium carbonate, sodium carbonate, potassium hydrogencarbonate and sodium hydrogencarbonate, and particularly preferred are potassium carbonate and sodium carbonate.

The molar ratio of the 3,5-dihalogeno-2-nitrobenzoic acid represented by the general formula (4) and the base, both used in the reaction, may be any molar ratio and the reaction proceeds satisfactorily. However, the proportion of the base is, for example, ordinarily 0.1 to 10.0 mols, preferably 0.33 to 3.0 mols, more preferably 1.0 to 2.5 mols relative to 1 mol of the 3,5-dihalogeno-2-nitrobenzoic acid represented by the general formula (4).

The reaction may be carried out in a solvent-free state. However, use of a solvent is preferred for smooth progress of the reaction. The solvent used in the reaction may be any solvent as long as it does not inhibit the reaction. As the solvent, there can be mentioned, for example, aprotic polar solvents such as acetone, methyl isobutyl ketone, N,N-dimethylformamide (DMF), N,N-dimethylacetamide (DMAC), acetonitrile, propylene carbonate and the like; alcohols such as methanol, ethanol, isopropanol, ethylene glycol and the like; ethers such as phenyl ether, diethyl ether, tetrahydrofuran (THF), dioxane and the like; aromatic hydrocarbons such as toluene, xylene, chlorobenzene and the like; halogen-containing solvents such as dichloromethane and the like; and aliphatic hydrocarbons such as pentane, n-hexane and the like. Preferred are aprotic polar solvents such as acetone, methyl isobutyl ketone, N,N-dimethylformamide (DMF), N,N-dimethylacetamide (DMAC), acetonitrile, propylene carbonate and the like. Particularly preferred are acetone and methyl isobutyl ketone.

The solvent may be used singly or in mixed solvent of any mixing proportions.

The amount of the solvent may be any amount as long as it allows for sufficient mixing of the reaction system. However, the amount is ordinarily 0 to 10 liters, preferably 0.05 to 10 liters, more preferably 0.2 to 2 liters relative to 1 mol of the 3,5-dihalogeno-2-nitrobenzoic acid compound represented by the general formula (4).

The temperature of the reaction is, for example, 0°C to the refluxing temperature of the solvent used, preferably 10 to 100°C.

The time of the reaction is not restricted particularly, but is preferably 1 hour to 30 hours in view of, for example, the prevention of by-product formation.

### Method B

The nitration of the alkyl 3,5-dihalogenobenzoate compound represented by the general formula (5) can be carried out by an ordinary conventional means using a nitrating agent. The nitrating agent used in the nitration may be any nitrating agent as long as it can give rise to the reaction. The nitrating agent may be used singly or in mixed agent of two or more kinds of any proportions.

As specific examples of the nitrating agent usable in the reaction, there can be mentioned nitric acid, concentrated nitric acid and fuming nitric acid and the like. As the nitrating agent and its concentration, there can be employed, for example, those described in SHIN JIKKEN KAGAKU KOUZA" Volume 14-III, 1261 to 1267 pages (published by Maruzen Co., Ltd. on Feb. 20, 1978). Specifically, there can be mentioned, for example, 20% nitric acid and 98% fuming nitric acid. Besides, there may be used alkali metal nitrates such as sodium nitrate, potassium nitrate and the like, nitronium trifluoromethanesulfonate, etc.

The molar ratio of the alkyl 3,5-dihalogenobenzoate compound represented by the general formula (5) and the nitrating agent, both used in the reaction, may be any molar ratio and the reaction proceeds satisfactorily. However, the proportion of the alkylating agent used is, for example, ordinarily 0.1 to 10.0 mols, preferably 0.33 to 3.0 mols, more preferably 1.0 to 2.0 mols relative to 1 mol of the alkyl 3,5-dihalogenobenzoate compound represented by the general formula (5).

The reaction may be carried out in a catalyst-free state, but may use a catalyst. As specific examples of the catalyst used in the reaction, there can be mentioned sulfuric acid, concentrated sulfuric acid and fuming sulfuric acid. The concentrations of these catalysts may be, for example, those described in the above-mentioned "SHIN JIKKEN KAGAKU KOUZA". Specifically, there can be mentioned, for example, 96% sulfuric acid and 30% fuming sulfuric acid (SO₃%). These catalysts may be used singly or in mixed catalyst of any proportions.

The molar ratio of the alkyl 3,5-dihalogenobenzoate compound represented by the general formula (5) and the catalyst, both used in the reaction, may be any molar ratio and the reaction proceeds satisfactorily. However, the proportion of the catalyst is, for example, ordinarily 0.1 to 10.0 mols, preferably 0.33 to 3.0 mols, more preferably 0.5 to 2.0 mols relative to 1 mol of the alkyl 3,5-dihalogenobenzoate compound represented by the general formula (5). However, when the catalyst functions also as a solvent, the catalyst may be used in a large excess, regardless of the above-mentioned use range.

The reaction may be carried out in a solvent-free state. However, use of a solvent is preferred for smooth progress of the reaction. The solvent used may be any solvent as long as it does not inhibit the reaction. There can be mentioned, for example, acidic solvents such as sulfuric acid, acetic acid, acetic anhydride, trifluoroacetic acid anhydride, trifluoromethanesulfonic acid and the like; ethers such as diethyl ether, tetrahydrofuran (THF), dioxane and the like; aromatic hydrocarbons such as chlorobenzene, o-dichlorobenzene, nitrobenzene and the like; halogen-containing solvents such as dichloromethane, chloroform, 1,2-dichloroethane, carbon tetrachloride and the like; aliphatic hydrocarbons such as hexane, heptane, cyclohexane and the like; and nitroalkanes such as nitromethane and the like. Preferably used are acidic solvents such as sulfuric acid, acetic acid, acetic anhydride, trifluoroacetic anhydride, trifluoromethanesulfonic acid and the like; and halogenated hydrocarbons such as dichloromethane, chloroform, 1,2-dichloroethane, carbon tetrachloride and the like; and dichloromethane is used particularly preferably.

The solvents may be used singly or in mixed solvent of any mixing proportions.

The amount of the solvent may be any amount as long as it allows for sufficient mixing of the reaction system. The amount may be ordinarily 0 to 10 liters, preferably 0.05 to 10 liters, more preferably 0.2 to 2 liters relative to 1 mol of the alkyl 3,5-dihalogenobenzoate compound represented by the general formula (5).

The temperature of the reaction is, for example, -20°C to 250°C, preferably 0 to 100°C.

The time of the reaction is not particularly restricted but, from the standpoint of, for example, the prevention of by-product formation, is preferably 10 minutes to 30 hours.

Description is made on the alkyl 3,5-dihalogeno-2-nitrobenzoate compound represented by the general formula (6), which is obtained as above from a 3,5-dihalogeno-2-nitrobenzoic acid compound represented by the general formula (4) or an alkyl 3,5-dihalogenobenzoate compound represented by the general formula (5).

In the general formula (6), the substituents R' and X are as defined above. Therefore, as specific examples of the alkyl 3,5-dihalogeno-2-nitrobenzoate compound represented by the general formula (6), there can be mentioned methyl 3,5-dichloro-2-nitrobenzoate, methyl 3,5-dibromo-2-nitrobenzoate, methyl 3,5-difluoro-2-nitrobenzoate, methyl 3,5-diiodo-2-nitrobenzoate, ethyl 3,5-dichloro-2-nitrobenzoate, ethyl 3,5-dibromo-2-nitrobenzoate, ethyl 3,5-difluoro-2-nitrobenzoate, ethyl 3,5-diiodo-2-nitrobenzoate, n-propyl 3,5-dichloro-2-nitrobenzoate, n-butyl 3,5-dibromo-2-nitrobenzoate, n-pentyl 3,5-difluoro-2-nitrobenzoate, isopropyl 3,5-diiodo-2-nitrobenzoate, and n-hexyl 3,5-dichlorobenzoate and the like.

Then, description is made on the process for producing a 2,4-dihalogeno-6-hydroxymethylnitrobenzene compound represented by the general formula (7). Incidentally, the substituent X in the general formula (7) is as defined above.

The 2,4-dihalogeno-6-hydroxymethylnitrobenzene represented by the general formula (7) can be produced by reducing an alkyl 3,5-dihalogeno-2-nitrobenzoate represented by the general formula (6).

The reaction uses a reducing agent. The reducing agent used in the reaction may be any reducing agent as long as it can give rise to the reaction. The reducing agent may be used singly or in mixed agent of two or more kinds, of any mixing proportions.

As specific examples of the reducing agent used in the reaction, there can be mentioned, for example, borohydride compounds such as sodium borohydride and the like; and aluminum hydride compounds such as lithium aluminum hydride and the like. Use of borohydride compound (e.g. sodium borohydride) is more preferred from the standpoints of availability, easy handling, reactivity, etc.

The molar ratio of the alkyl 3,5-dihalogeno-2-nitrobenzoate compound represented by the general formula (6) and the reducing agent, both used in the reaction, may be any molar ratio and the reaction proceeds satisfactorily. However, the proportion of the reducing agent is, for example, ordinarily 0.1 to 10.0 mols, preferably 0.33 to 3.0 mols, more preferably 0.5 to 2.0 mols relative to 1 mol of the alkyl 3,5-dihalogeno-2-nitrobenzoate compound represented by the general formula (6).

In the reaction, use of an alcohol is preferred when a borohydride compound is used. As specific examples of the alcohol used in the reaction, there can be mentioned C1 to C6 alkanols represented by methanol, ethanol, propanol, isopropanol and butanol; and alkanediols represented by ethylene glycol. These alcohols may be used singly or in mixed alcohol of two or more kinds of any proportions. Methanol and ethanol are preferred from the standpoints of availability, easy handling, reactivity, etc., and methanol is more preferred.

The molar ratio of the alkyl 3,5-dihalogeno-2-nitrobenzoate compound represented by the general formula (6) and the alcohol, both used in the reaction, may be any molar ratio and the reaction proceeds satisfactorily. However, the proportion of the alcohol is, for example, ordinarily 0.2 to 20.0 mols, preferably 0.66 to 6.0 mols, more preferably 1.0 to 4.0 mols relative to 1 mol of the alkyl 3,5-dihalogeno-2-nitrobenzoate compound represented by the general formula (6). However, when the alcohol functions also as a solvent (described later), the alcohol may be used in a large excess, regardless of the above-mentioned use range.

The reaction may be carried out in a solvent-free state. However, use of a solvent is preferred for smooth progress of the reaction. The solvent used in the reaction may be any solvent as long as it does not inhibit the reaction. There can be mentioned, for example, aprotic polar solvents such as N,N-dimethylformamide (DMF), N,N-dimethylacetamide (DMAC), acetonitrile, propylene carbonate and the like; ethers such as phenyl ether, diethyl ether, tetrahydrofuran (THF), dioxane and the like; aromatic hydrocarbons such as toluene, xylene, chlorobenzene and the like; halogen-containing solvents such as dichloromethane and the like; aliphatic hydrocarbons such as pentane, n-hexane and the like; and alcohols such as methanol, ethanol, propanol, isopropanol, butanol and the like. Preferably used are ethers such as phenyl ether, diethyl ether, tetrahydrofuran (THF), dioxane and the like; or alcohols such as methanol, ethanol, propanol, isopropanol, butanol and the like. Particularly preferably used is tetrahydrofuran (THF) or methanol.

The above solvents may be used singly or in mixed solvent of two or more kinds of any proportions.

The amount of the solvent may be any amount as long as it allows for sufficient mixing of the reaction system. However, the amount is ordinarily 0 to 10 liters, preferably 0.05 to 10 liters, more preferably 0.2 to 2 liters relative to 1 mol of the alkyl 3,5-dihalogeno-2-nitrobenzoate compound represented by the general formula (6).

The temperature of the reaction is, for example, 0°C to the refluxing temperature of the solvent used, but is preferably 10 to 100°C.

The time of the reaction is not particularly restricted but is preferably 1 hour to 30 hours from the standpoint of, for example, the prevention of by-product formation.

The 2,4-dihalogeno-6-hydroxymethylnitrobenzene represented by the general formula (7), obtained as above is a novel compound. Specifically, there can be mentioned 2,4-dichloro-6-hydroxymethylnitrobenzene, 2,4-dibromo-6-hydroxymethylnitrobenzene, 2,4-difluoro-6-hydroxymethylnitrobenzene, and 2,4-diiodo-6-hydroxymethylnitrobenzene.

Next, description is made on the process for producing a 2,4-dihalogenonitrobenzene derivative represented by the general formula (1).

The 2,4-dihalogenonitrobenzene compound represented by the general formula (1) is a novel compound which can be produced by subjecting the 2,4-dihalogeno-6-hydroxymethylnitrobenzene compound represented by the general formula (7), obtained as above, to alkylation.

The reaction uses an alkylating agent. The alkylating agent used in the reaction may be any alkylating agent as long as it can give rise to the reaction. The alkylating agent may be used singly or in mixed agent of two or more kinds of any proportions. A specific examples of the alkylating agent, there can be mentioned alkylating agents capable of introducing a "C1 to C6 alkyl group", such as dialkyl sulfates (represented by dimethyl sulfate, diethyl sulfate, di-n-propyl sulfate and diisopropyl sulfate), alkyl alkylsulfonates and alkyl arylsulfonates (represented by methyl methanesulfonate, methyl toluenesulfonate, ethyl methanesulfonate, ethyl toluenesulfonate, n-propyl methanesulfonate, n-propyl toluenesulfonate, isopropyl methanesulfonate, isopropyl toluenesulfonate, n-butyl methanesulfonate, n-pentyl methanesulfonate and n-hexyl methanesulfonate), alkyl halides (represented by methyl iodide, methyl bromide, methyl chloride, ethyl iodide, ethyl bromide, ethyl chloride, propyl iodide, propyl bromide, propyl chloride, isopropyl iodide, isopropyl bromide, isopropyl chloride, butyl bromide, pentyl bromide, hexyl bromide, etc), alcohols (represented by methanol, ethanol, n-propanol and isopropanol), and the like. As specific examples of the alkyl group introduced, there can be mentioned methyl group, ethyl group, n-propyl group, isopropyl group, n-butyl group, isobutyl group, n-pentyl group and n-hexyl group. Preferably used are dialkyl sulfates, alkyl alkylsulfonates and alkyl halides from the standpoints of availability, easy handling, reactivity, etc. More preferred are dimethyl sulfate, diethyl sulfate, methyl methanesulfonate, ethyl bromide, propyl bromide, isopropyl bromide and butyl bromide.

The molar ratio of the 2,4-dihalogeno-6-hydroxymethylnitrobenzene compound represented by the general formula (7) and the alkylating agent, both used in the reaction, may be any molar ratio and the reaction proceeds satisfactorily. However, the proportion of the alkylating agent is, for example, ordinarily 0.1 to 10.0 mols, preferably 0.33 to 3.0 mols, more preferably 1.0 to 2.0 mols relative to 1 mol of the 2,4-dihalogeno-6-hydroxymethylnitrobenzene represented by the general formula (7).

The reaction may be carried out in a catalyst-free state. However, a phase transfer catalyst is preferably used for smooth progress of the reaction. As specific examples of the phase transfer catalyst used in the reaction, there can be mentioned quaternary ammonium salts such as tetra-n-butylammonium bromide (TBAB) and the like; and cyclic ethers (crown ethers) such as 18-crown-6 and the like. These phase transfer catalysts may be used singly or in mixed catalyst of any proportions. Quaternary ammonium salts such as tetra-n-butylammonium bromide (TBAB) and the like are preferably used from the standpoints of availability, easy handling, reactivity, etc., and tetra-n-butylammonium bromide (TBAB) is used more preferably.

The molar ratio of the 2,4-dihalogeno-6-hydroxymethylnitrobenzene compound represented by the general formula (7) and the phase transfer catalyst, both used in the reaction, may be any molar ratio and the reaction proceeds satisfactorily. However, the proportion of the phase transfer catalyst is, for example, ordinarily 0 to 1.0 mol, preferably 0.001 to 1.0 mol, more preferably 0.01 to 0.5 mol, particularly preferably 0.02 to 0.1 mol relative to 1 mol of the 2,4-dihalogeno-6-hydroxymethylnitrobenzene compound represented by the general formula (7).

The reaction proceeds in the presence of a base. As specific examples of the base, there can be mentioned inorganic bases such as alkali metal hydroxides (represented by sodium hydroxide, potassium hydroxide and lithium hydroxide); alkali metal carbonates (represented by potassium carbonate and sodium carbonate); alkali metal hydrogencarbonates (represented by potassium hydrogencarbonate and sodium hydrogencarbonate) and the like; aqueous solutions of the inorganic bases; and organic bases such as pyridine, tertiary amines (e.g. N,N-diisopropylethylamine and triethylamine) and the like. These bases may be used singly or in mixed base of any proportions. Preferably used are aqueous solutions of inorganic bases such as sodium hydroxide, potassium hydroxide, potassium carbonate, sodium carbonate, potassium hydrogencarbonate, sodium hydrogencarbonate and the like; more preferably used is an aqueous sodium hydroxide solution.

The molar ratio of the 2,4-dihalogeno-6-hydroxymethylnitrobenzene compound represented by the general formula (7) and the base, both used in the reaction, may be any molar ratio and the reaction proceeds satisfactorily. However, the proportion of the base is, for example, ordinarily 0.1 to 10.0 mols, preferably 0.33 to 3.0 mols, more preferably 1.0 to 2.0 mols relative to 1 mol of the 2,4-dihalogeno-6-hydroxymethylnitrobenzene compound represented by the general formula (7).

The reaction may be carried out in a solvent-free state. However, a solvent is used preferably for smooth progress of the reaction. The solvent used in the reaction may be any solvent as long as it does not inhibit the reaction. There can be mentioned, for example, aromatic hydrocarbons such as toluene, xylene, chlorobenzene and the like; halogen-containing solvents such as dichloromethane and the like; ethers such as phenyl ether, diethyl ether, tetrahydrofuran (THF), dioxane and the like; and aliphatic hydrocarbons such as pentane, n-hexane and the like. Preferably used are aromatic hydrocarbons such as toluene, xylene, chlorobenzene and the like; more preferably used is toluene.

The solvent may be used singly or in mixed solvent of two or more kinds of any proportions.

The amount of the solvent may be any amount as long as it allows for sufficient mixing of the reaction system. However, the amount is ordinarily 0 to 10 liters, preferably 0.05 to 10 liters, more preferably 0.2 to 2 liters relative to 1 mol of the 2,4-dihalogeno-6-hydroxymethylnitrobenzene compound represented by the general formula (7).

The temperature of the reaction is, for example, 0°C to the refluxing temperature of the solvent used, but is preferably 0 to 100°C.

The time of the reaction is not particularly restricted but is preferably 1 hour to 30 hours from the standpoint of, for example, the prevention of by-product formation.

The 2,4-dihalogenonitrobenzene compound represented by the general formula (1), obtained as above is a novel compound which can be produced from a 3,5-dihalogeno-2-nitrobenzoic acid compound represented by the general formula (4), or from an alkyl 3,5-dihalogenobenzoate compound represented by the general formula (5), both of good availability. Since the 2,4-dihalogenonitrobenzene compound represented by the general formula (1) can be easily derived from a raw material of good availability, it has become possible to easily produce a pyrimidinylacetonitrile derivative represented by the general formula (3) by reacting the 2,4-dihalogenonitrobenzene compound represented by the general formula (1) with 4,6-dimethoxy-2-cyanomethylpyridine represented by formula (2).

According to the present invention, an intended pyrimidinylacetonitrile derivative can be produced at a high selectivity, efficiently, in an easy operation, and under mild conditions, without using any special reactor, by using, as a raw material, a 3,5-dihalogeno-2-nitrobenzoic acid compound or an alkyl 3,5-dihalogenobenzoate compound, both of good availability. Further, since the 3,5-dihalogeno-2-nitrobenzoic acid compound and the alkyl 3,5-dihalogenobenzoate compound can be produced from a 3,5-dihalogenobenzoic acid which is a symmetric substance of simple structure, the process makes it possible to selectively produce, at a high yield, a pyrimidinylacetonitrile derivative having phenyl group of asymmetric structure, e.g. (5-chloro-3-methoxymethyl-2-nitrophenyl)(4,6-dimethoxypyrimidin-2-yl)acetonitrile. The obtained pyrimidinylacetonitrile derivative represented by the general formula (3) is a compound useful as an intermediate for herbicide synthesis.

### EXAMPLES

Next, the process for producing the compound of the present invention is described specifically by way of Examples. However, the present invention is in no way restricted by the Examples.

### Example 1 Production of methyl 3,5-dichloro-2-nitrobenzoate

Into a 1-liter, four-necked flask equipped with a mechanical stirrer, a thermometer and a reflux condenser were added 129.0 g (547 mmol) of 3,5-dichloro-2-nitrobenzoic acid, 76 g (550 mmol) of potassium carbonate, 550 ml of acetone and 76.3 g (600 mmol) of dimethyl sulfate, followed by stirring at 50°C for 5 hours. Acetone was recovered under reduced pressure. Then, 400 ml of ethyl acetate and 500 ml of water were added for phase separation. Re-extraction was conducted with 200 ml of ethyl acetate. The ethyl acetate phases were washed with water and a saturated aqueous sodium chloride solution in this order and dried over anhydrous sodium sulfate. Then, ethyl acetate was distilled off under reduced pressure to obtain 148.8 g of a light yellow oil. To the oil were added 300 ml of diisopropyl ether and 150 ml of n-hexane to give rise to crystallization. The crystal obtained was filtered and washed with 300 ml of n-hexane to obtain 111.7 g of a white crystal. Crystallization was made two more times using the residue of the filtrate, to obtain methyl 3,5-dichloro-2-nitrobenzoate as 19.4 g of a white crystal. HPLC purity = >99.8% Yield = 96%

### Example 2 Production of methyl 3,5-dichloro-2-nitrobenzoate

Into a 300-ml, four-necked flask equipped with a mechanical stirrer, a thermometer and a reflux condenser were added 41.0 g (200 mmol) of methyl 3,5-dichlorobenzoate and 100 ml of dichloromethane. Thereto was added dropwise 15.4 g (240 mmol) of fuming nitric acid, in an ice bath. Then, 22.4 g of 30% fuming sulfuric acid (SO₃ %) (containing 160 mmol of H₂SO₄) was added dropwise in an ice bath. Stirring was conducted at room temperature for 2 hours. The reaction mixture was added dropwise into an ice of 200 ml for phase separation. The dichloromethane phase was washed with a saturated aqueous sodium hydrogen carbonate solution, to obtain methyl 3,5-dichloro-2-nitrobenzoate in a state dissolved in the dichloromethane phase. The yield obtained from a GC internal standard method (internal standard substance: dodecane) was 97.0% and the HPLC purity was 95.4%.

### Melting point: 71 to 72°C

¹H NMR (300 MHz, CDCl₃) δ value:
7.96 (d, J=2.1 Hz, 1H), 7.71 (d, J=2.1 Hz, 1H),
3.93 (s, 3H) ppm
¹³C NMR (300 MHz, CDCl₃) δ value:
161.8, 136.8, 134.4, 130.0, 127.6, 125.7, 53.9 ppm GC-MS M⁺ = 249

### Example 3 Production of 2,4-dichloro-6-hydroxymethylnitrobenzene

Into a 100-ml, eggplant-shaped flask equipped with a magnetic stirrer and a dropping funnel were added 3.6 g (14.4 mmol) of methyl 3,5-dichloro-2-nitrobenzoate, 0.65 g (17.3 mmol) of sodium borohydride and 8 ml of THF. Thereto was added dropwise 0.92 g (28.8 mmol) of methanol over 30 minutes, with refluxing. Then, stirring was conducted for 3 hours. THF was recovered under reduced pressure. Then, 50 ml of ethyl acetate and 50 ml of 2% hydrochloric acid were added for phase separation. The ethyl acetate phase was washed with water and a saturated aqueous sodium chloride solution in this order, and dried over anhydrous sodium sulfate. Ethyl acetate was distilled off under reduced pressure to obtain 3.4 g of a light brown crude crystal. HPLC purity = 95.2% Crude yield = 107%. The light brown crude crystal was purified by silica gel column chromatography (ethyl acetate-hexane) and then recrystallized from toluene-hexane to obtain 2.1 g of 2,4-dichloro-6-hydroxymethylnitrobenzene as a white crystal. HPLC purity = 99.8% Yield = 65.7%

### Melting point: 133 to 134°C

¹H NMR (300 MHz, CDCl₃) δ value:
7.56 (d, J=2.1 Hz, 1H), 7.48 (d, J=2.1 Hz, 1H),
4.71 (s, 2H) ppm
GC-MS M⁺ = 221

### Example 4 Production of 2,4-dichloro-6-methoxymethylnitrobenzene

Into a 100-ml, eggplant-shaped flask equipped with a magnetic stirrer and a dropping funnel were added 2.22 g (10 mmol) of a crude crystal of 2,4-dichloro-6-hydroxymethylnitrobenzene, 10 ml of toluene and 0.64 g (1 mmol) of 50% tetra-n-butylammonium bromide (TBAB). Thereto was added 2.4 g (15 mmol) of a 25% aqueous sodium hydroxide solution in an ice bath. 1.89 g (15 mmol) of dimethyl sulfate was added dropwise in an ice bath. Stirring was conducted at room temperature for 2.5 hours. To the system were added 75 ml of water and 50 ml of toluene for phase separation. Re-extraction was conducted using 30 ml of toluene. The toluene phases were washed with water and a saturated aqueous sodium chloride solution in this order and dried over anhydrous sodium sulfate. Toluene was distilled off under reduced pressure to obtain 2,4-dichloro-6-methoxymethylnitrobenzene as 2.33 g of a red oily crude product. HPLC purity = 90.3% Crude yield = 98.7%

¹H NMR (300 MHz, CDCl₃) δ value:
7.48 (d, J=1.8 Hz, 1H), 7.46 (d, J=1.8 Hz, 1H),
4.45 (s, 2H), 3.40 (s, 3H) ppm
GC-MS M⁺ = 235

### Example 5 Production of (5-chloro-3-methoxymethyl-2-nitrophenyl)(4,6-dimethoxypyrimidin-2-yl)-acetonitrile

Into a 500-ml, four-necked flask equipped with a mechanical stirrer and a thermometer were added 71 g (corresponding to 300 mmol) of crude 2,4-dichloro-6-methoxymethylnitrobenzene, 53.8 g (300 mmol) of 2-cyanomethyl-4,6-dimethoxypyrimidine and 150 ml of DMF. The mixture was stirred until it became a homogeneous solution. Thereto was added 26.4 g (660 mmol) of bead-like sodium hydroxide in an ice bath, and the whole mixture was stirred for 4 hours while the temperature was gradually returned to room temperature. To the reaction system were added 640 ml of 2% hydrochloric acid and 300 ml of ethyl acetate for phase separation. The aqueous phase was subjected to re-extraction with 200 ml of ethyl acetate. The ethyl acetate phases obtained were washed with water and a saturated aqueous sodium chloride solution in this order and dried over anhydrous sodium sulfate. Ethyl acetate was distilled off under reduced pressure to obtain 115.3 g of a brown crude crystal. HPLC purity = 88.2% Crude yield = 101.5% Of the crude crystal obtained, 40 g was recrystallized from methanol to obtain 27.5 g of a light brown crystal (HPLC purity: 99.7%). The residue of the filtrate was subjected to re-crystallization from methanol to obtain (5-chloro-3-methoxymethyl-2-nitrophenyl)(4,6-dimethoxypyrimidin-2-yl)acetonitrile as 5 g of a light yellow crystal (HPLC purity: 97.0%) . Yield = 80%

### Melting point: 85 to 87°C

¹H NMR (300 MHz, CDCl₃) δ value:
7.81 (d, J=2.4 Hz, 1H), 7.63 (d, J=2.4 Hz, 1H),
5.93 (s, 1H), 5.74 (s, 1H), 4.51 (ABq, J_{AB}=13.5 Hz, 2H),
3.90 (s, 6H), 3.42 (s, 3H) ppm
LC-MS (M+1)⁺ = 379.1

### (Reference Example 1)

### Production of 3,5-dichloro-2-nitrobenzoic acid

Into a 500-ml, four-necked flask equipped with a mechanical stirrer, a dropping funnel and a thermometer were added 490 g (5 mol) of concentrated sulfuric acid and 95.5 g (0.5 mol) of 3,5-dichlorobenzoic acid. Thereto was added dropwise 37.8 g (d ₌ 1.52, 0.6 mol) of concentrated nitric acid with the temperature of the system being controlled to be 20°C or lower. Then, stirring was conducted at room temperature for 2.5 hours. The reaction mixture was poured into 1,000 g of ice, followed by stirring and filtration. The crystal obtained was washed with 2 liters of water and dried to obtain 3,5-dichloro-2-nitrobenzoic acid as 112.4 g of a white crystal. HPLC purity = 96.8% Yield = 95.3%

¹H NMR (300 MHz, CDCl₃) δ value:
8.02 (d, J=2.1 Hz, 1H), 7.76 (d, J=2.1 Hz, 1H) ppm GC-MS M⁺ = 235

### (Reference Example 2)

### Production of (5-chloro-3-methoxymethyl-2-nitrophenyl) (4,6-dimethoxypyrimidin-2-yl)ketone

Into a 200-ml, four-necked flask equipped with a mechanical stirrer, a dropping funnel and a thermometer were added 26.5 g (corresponding to 70 mmol) of crude (5-chloro-3-methoxymethyl-2-nitrophenyl) (4,6-dimethoxypyrimidin-2-yl)acetonitrile and 70 ml of DMF. They were made into a solution. Thereto was added 11.2 g (70 mmol) of a 25% aqueous sodium hydroxide solution with the temperature of the system being controlled to be 10°C or lower. 20.4 g (210 mmol) of 35% hydrogen peroxide was added dropwise with the system temperature being controlled to be 10°C or lower. Then, stirring was conducted at room temperature for 2.5 hours. To the reaction mixture were added 200 ml of water, 200 ml of ethyl acetate and about 50 g of 35% hydrochloric acid for phase separation. Re-extraction was made with 200 ml of ethyl acetate. The ethyl acetate phases were combined, washed with water and a saturated aqueous sodium chloride solution in this order, and dried over anhydrous sodium sulfate. Ethyl acetate was distilled off under reduced pressure to obtain 27.5 g of a crude crystal. 70 ml of diisopropyl ether was added to the crude crystal to obtain a suspension. The suspension was filtered to obtain (5-chloro-3-methoxymethyl-2-nitrophenyl)(4,6-dimethoxypyrimidin-2-yl)ketone as a light brown crystal. HPLC purity = 99.3% Yield = 77.7%

### Melting point: 130 to 131°C

¹H NMR (300 MHz, CDCl₃) δ value:
7.90 (d, J=2.4 Hz, 1H), 7.54 (d, J=2.4 Hz, 1H),
6.14 (s, 1H), 4.75 (s, 2H), 3.89 (s, 6H), 3.50 (s, 3H) • ppm
LC-MS (M+1)⁺ = 368.0

### (Reference Example 3)

### Production of (5-chloro-3-methoxymethyl-2-nitrophenyl) (4,6-dimethoxypyrimidin-2-yl)ketone

Into a 50-ml, eggplant-shaped flask equipped with a mechanical stirrer and a dropping funnel were added 2.36 g (corresponding to 10 mmol) of crude 2,4-dichloro-6-methoxymethylnitrobenzene, 1.79 g (10 mmol) of 2-cyanomethyl-4,6-dimethoxypyrimidine and 10 ml of DMF. Stirring was conducted until the mixture became a homogeneous solution. Thereto was added 0.84 g (21 mmol) of bead-like sodium hydroxide in an ice bath, and stirring was conducted for 4 hours with the temperature being slowly returned to room temperature. HPLC analysis was conducted to confirm the formation of (5-chloro-3-methoxymethyl-2-nitrophenyl)(4,6-dimethoxypyrimidin-2-yl)acetonitrile. Then, to the system was added dropwise 2.92 g (30 mmol) of 35% hydrogen peroxide with the temperature being controlled to be 10°C or lower, after which stirring was conducted at room temperature for 3 hours. The reaction mixture was poured into an aqueous solution obtained by adding 2 g (20 mmol) of 35% hydrochloric acid to 50 ml of water, followed by sufficient stirring, filtered and washed with 30 ml of water. The crystal obtained was dried to obtain (5-chloro-3-methoxymethyl-2-nitrophenyl)(4,6-dimethoxypyrimidin-2-yl)ketone as 3.42 g of a light brown crystal. HPLC purity = 95.5% Yield = 93.0%

LC-MS (M+1)⁺= 368.0

### INDUSTRIAL APPLICABILITY

The present invention provides a novel process for industrial production of a pyrimidinylacetonitrile derivative. According to the present process, an intended pyrimidinylacetonitrile derivative can be produced at a high selectivity, in an easy operation, and under mild conditions without using any special reactor, or any expensive catalyst or transition metal, by using, as a raw material, a 3,5-dihalogeno-2-nitrobenzoic acid compound represented by the general formula (4) or an alkyl 3,5-dihalogenobenzoate compound represented by the general formula (5), both of good availability. Moreover, since there is no harmful waste substance derived from catalyst or transition metal, the present process is easy in waste disposal and friendly to the environment. Thus, the present invention has high industrial applicability.

## Claims

1. A process for producing a pyrimidinylacetonitrile derivative represented by the general formula (3) 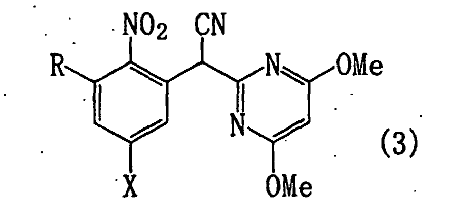 (wherein X is a halogen atom, R is an alkoxymethyl group, and Me is a methyl group), **characterized by** reacting, in the presence of a base, a 2,4-dihalogenonitrobenzene compound represented by the general formula (1) 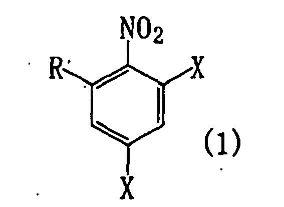 (wherein X and R are as defined above) with 4,6-dimethoxy-2-cyanomethylpyrimidine represented by a formula (2) 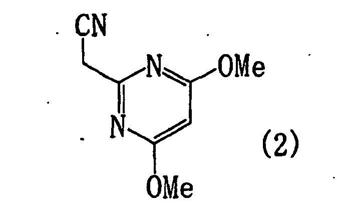 (wherein Me is as defined above).

2. A process for producing a pyrimidinylacetonitrile derivative set forth in Claim 1, wherein the 2,4-dihalogenonitrobenzene compound represented by the general formula (1) 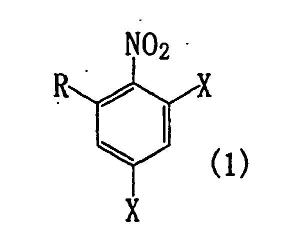 (wherein X is a halogen atom, and R is an alkoxymethyl group) is produced by subjecting a 3,5-dihalogeno-2-nitrobenzoic acid compound represented by the general formula (4) 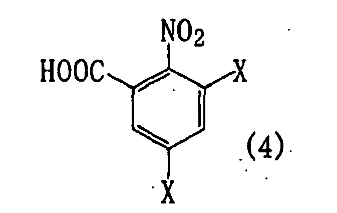 (wherein X is as defined above) to alkylation in the presence of a base (method A) or subjecting an alkyl 3,5-dihalogenobenzoate compound represented by the general formula (5) 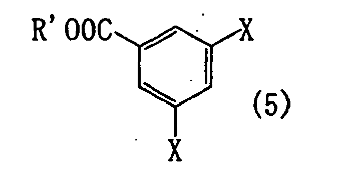 (wherein R' is an alkyl group, and X is as defined above) to nitration (method B), to obtain an alkyl 3,5-dihalogeno-2-nitrobenzoate compound represented by the general formula (6) 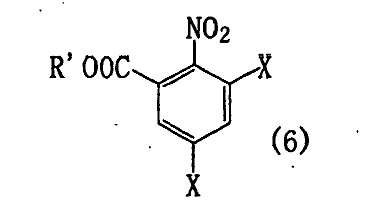 (wherein X and R' are as defined above), then reducing the alkyl 3,5-dihalogeno-2-nitrobenzoate compound to obtain a 2,4-dihalogeno-6-hydroxymethylnitrobenzene compound represented by the general formula (7) 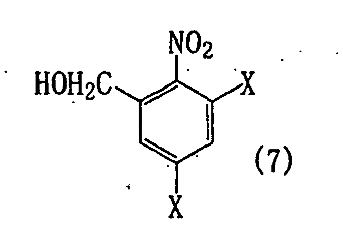 (wherein X is as defined above), and subjecting the 2,4-dihalogeno-6-hydroxymethylnitrobenzene compound to alkylation in the presence of a base.

3. A process for producing a pyrimidinylacetonitrile derivative set forth in Claim 2, wherein the alkyl 3,5-dihalogeno-2-nitrobenzoate compound represented by the general formula (6) 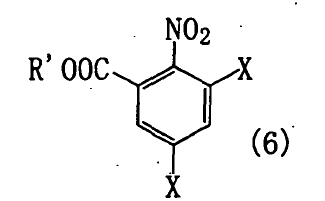 (wherein R' is an alkyl group, and X is a halogen atom) is produced by subjecting a 3,5-dihalogeno-2-nitrobenzoic acid compound represented by the general formula (4) 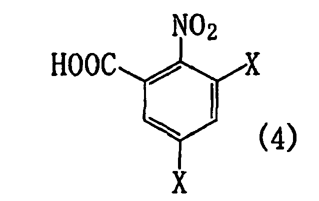 (wherein X is as defined above) to alkylation in the presence of a base (method A).

4. A process for producing a pyrimidinylacetonitrile derivative set forth in Claim 2, wherein the alkyl 3,5-dihalogeno-2-nitrobenzoate compound represented by the general formula (6) 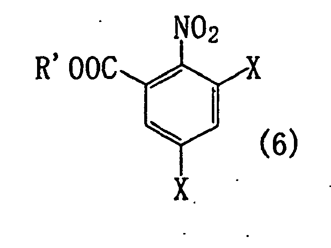 (wherein R' is an alkyl group, and X is a halogen atom) is produced by subjecting an alkyl 3,5-dihaogenobenzoate compound represented by the general formula (5) 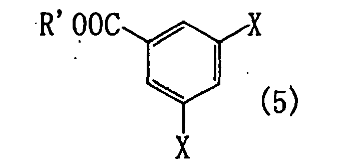 (wherein R' and X are as defined above) to nitration (method B).

5. A process for producing a pyrimidinylacetonitrile derivative set forth in Claim 1, wherein the 2,4-dihalogenonitrobenzene compound represented by the general formula (1) 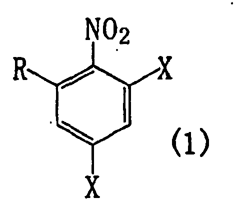 (wherein X is a halogen atom, and R is an alkoxymethyl group) is produced by reducing an alkyl 3,5-dihalogeno-2-nitrobenzoate compound represented by the general formula (6) 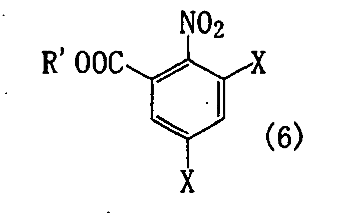 (wherein R' is an alkyl group, and X is a halogen atom) to obtain a 2,4-dihalogeno-6-hydroxymethylnitrobenzene compound represented by the general formula (7) 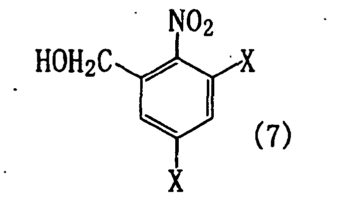 (wherein X is as defined above), and subjecting the 2,4-dihalogeno-6-hydroxymethylnitrobenzene compound to alkylation in the presence of a base.

6. A 2,4-dihalogenonitrobenzene compound represented by the general formula (1) 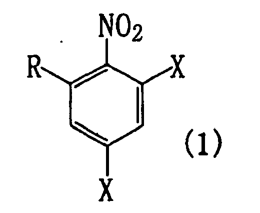 (wherein X is a halogen atom, and R is an alkoxymethyl group).

7. A 2,4-dihalogeno-6-hydroxymethylnitrobenzene compound represented by the general formula (7) 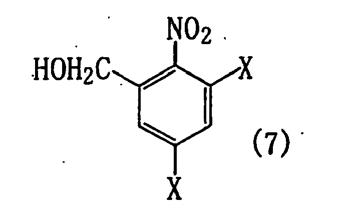 (wherein X is a halogen atom).

## Patentansprüche

1. Ein Verfahren zur Herstellung eines Pyrimidinylacetonitrilderivats, dargestellt durch die allgemeine Formel (3), 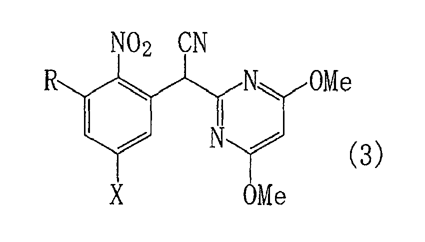 (wobei X ein Halogenatom ist, R ein Alkoxymethylrest ist und Me ein Methylrest ist), **gekennzeichnet durch** Umsetzen, in Gegenwart einer Base, einer 2,4-Dihalogennitrobenzolverbindung, dargestellt **durch** die allgemeine Formel (1), 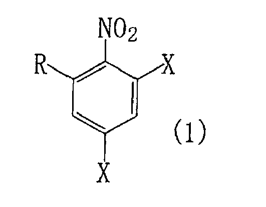 (wobei X und R wie vorstehend definiert sind) mit 4,6-Dimethoxy-2-cyanomethylpyrimidin, dargestellt **durch** eine Formel (2), 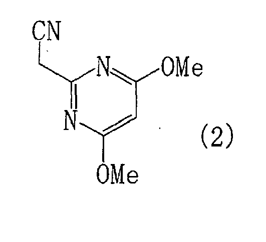 (wobei Me wie vorstehend definiert ist).

2. Ein Verfahren zur Herstellung eines Pyrimidinylacetonitrilderivats wie in Anspruch 1 dargelegt, wobei die 2,4-Dihalogennitrobenzolverbindung, dargestellt durch die allgemeine Formel (1), 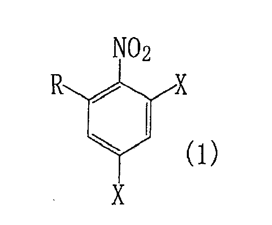 (wobei X ein Halogenatom ist und R ein Alkoxymethylrest ist) hergestellt wird durch Unterziehen einer 3,5-Dihalogen-2-nitrobenzoesäureverbindung, dargestellt durch die allgemeine Formel (4), 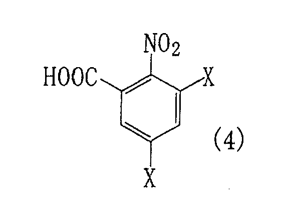 (wobei X wie vorstehend definiert ist) einer Alkylierung in Gegenwart einer Base (Verfahren A) oder Unterziehen einer 3,5-Dihalogenbenzoesäurealkylesterverbindung, dargestellt durch die allgemeine Formel (5), 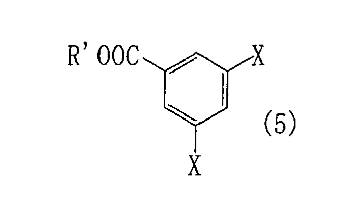 (wobei R' ein Alkylrest ist und X wie vorstehend definiert ist) einer Nitrierung (Verfahren B), um eine 3,5-Dihalogen-2-nitrobenzoesäurealkyesterverbindung, dargestellt durch die allgemeine Formel (6), 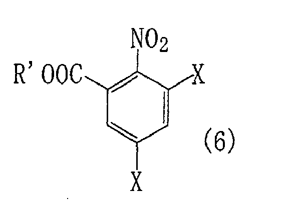 zu erhalten (wobei X und R' wie vorstehend definiert sind), dann Reduzieren der 3,5-Dihalogen-2-nitrobenzoesäurealkyesterverbindung, um eine 2,4-Dihalogen-6-hydroxymethylnitrobenzolverbindung, dargestellt durch die allgemeine Formel (7), 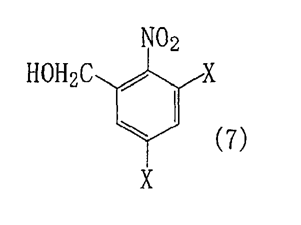 zu erhalten (wobei X wie vorstehend definiert ist) und Unterziehen der 2,4-Dihalogen-6-hydroxymethylnitrobenzolverbindung einer Alkylierung in Gegenwart einer Base.

3. Ein Verfahren zur Herstellung eines Pyrimidinylacetonitrilderivats wie in Anspruch 2 dargelegt, wobei die 3,5-Dihalogen-2-nitrobenzoesäurealkyesterverbindung, dargestellt durch die allgemeine Formel (6), 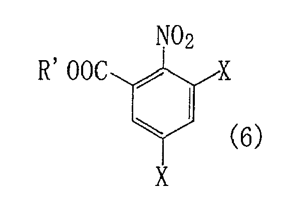 (wobei R' ein Alkylrest ist und X ein Halogenatom ist) hergestellt wird durch Unterziehen einer 3,5-Dihalogen-2-nitrobenzoesaureverbindung, dargestellt durch die allgemeine Formel (4), 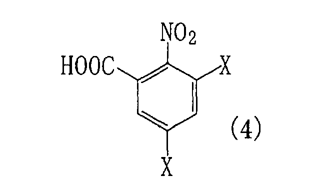 (wobei X wie vorstehend definiert ist) einer Alkylierung in Gegenwart einer Base (Verfahren A).

4. Ein Verfahren zur Herstellung eines Pyrimidinylacetonitrilderivats wie in Anspruch 2 dargelegt, wobei die 3,5-Dihalogen-2-nitrobenzoesäurealkyesterverbindung, dargestellt durch die allgemeine Formel (6), 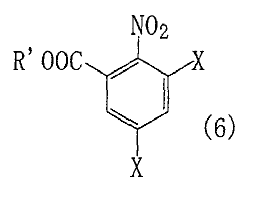 (wobei R' ein Alkylrest ist und X ein Halogenatom ist) hergestellt wird durch Unterziehen einer 3,5-Dihalogenbenzoesaurealkylesterverbindung, dargestellt durch die allgemeine Formel (5), 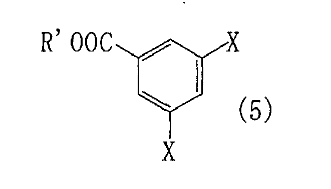 (wobei R' und X wie vorstehend definiert sind) einer Nitrierung (Verfahren B).

5. Ein Verfahren zur Herstellung eines Pyrimidinylacetonitrilderivats wie in Anspruch 1 dargelegt, wobei die 2,4-Dihalogennitrobenzolverbindung, dargestellt durch die allgemeine Formel (1), 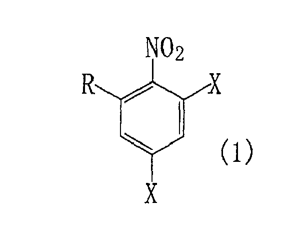 (wobei X ein Halogenatom ist und R ein Alkoxymethylrest ist) hergestellt wird durch Reduzieren einer 3,5-Dihalogen-2-nitrobenzoesäurealkyesterverbindung, dargestellt durch die allgemeine Formel (6), 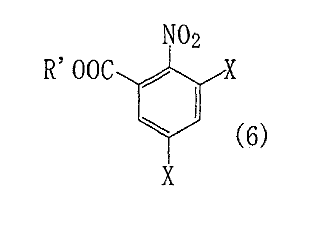 (wobei R' ein Alkylrest ist und X ein Halogenatom ist), um eine 2,4-Dihalogen-6-hydroxymethylnitrobenzolverbindung, dargestellt durch die allgemeine Formel (7), 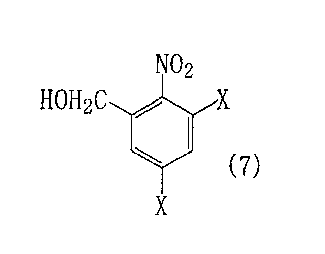 zu erhalten (wobei X wie vorstehend definiert ist) und Unterziehen der 2,4-Dihalogen-6-hydroxymethylnitrobenzolverbindung einer Alkylierung in Gegenwart einer Base.

6. Eine 2,4-Dihalogennitrobenzolverbindung, dargestellt durch die allgemeine Formel (1), 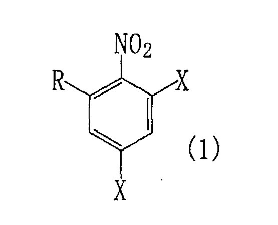 (wobei X ein Halogenatom ist und R ein Alkoxymethylrest ist).

7. Eine 2,4-Dihalogen-6-hydroxymethylnitrobenzolverbindung, dargestellt durch die allgemeine Formel (7), 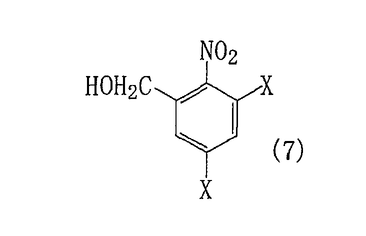 (wobei X ein Halogenatom ist).

## Revendications

1. Procédé de production d'un dérivé de pyrimidinylacétonitrile représenté par la formule générale (3) 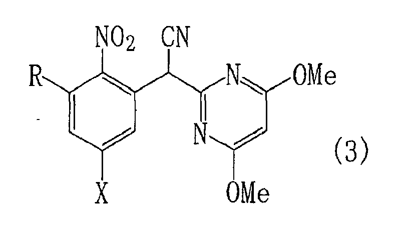 (dans laquelle X représente un atome d'halogène, R représente un groupe alcoxyméthyle, et Me représente un groupe méthyle), **caractérisé par** la réaction, en présence d'une base, d'un composé de 2,4-dihalogénonitrobenzène représenté par la formule générale (1) 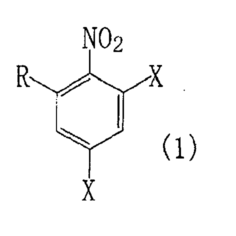 (dans laquelle X et R sont tels que définis ci-dessus) avec de la 4,6-diméthoxy-2-cyanométhylpyrimidine représentée par une formule (2) 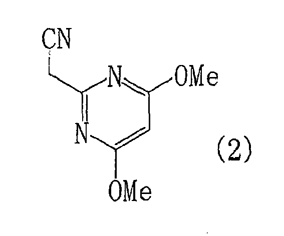 (dans laquelle Me est tel que défini ci-dessus).

2. Procédé de production d'un dérivé de pyrimidinylacétonitrile présenté dans la revendication 1, dans lequel le composé de 2,4-dihalogénonitrobenzène représenté par la formule générale (1) 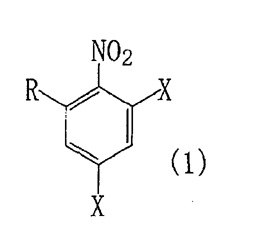 (dans laquelle X représente un atome d'halogène, et R représente un groupe alcoxyméthyle) est produit en soumettant un composé d'acide 3,5-dihalogéno-2-nitrobenzoïque représenté par la formule générale (4) 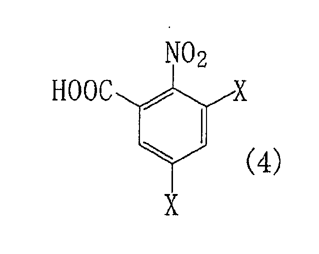 (dans laquelle X est tel que défini ci-dessus) à une alkylation en présence d'une base (méthode A) ou en soumettant un composé de 3,5-dihalogénobenzoate d'alkyle représenté par la formule générale (5) 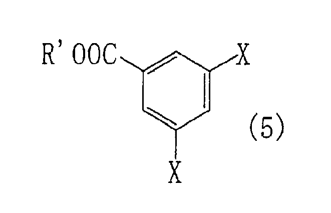 (dans laquelle R' représente un groupe alkyle, et X est tel que défini ci-dessus) à une nitration (méthode B), pour obtenir un composé de 3,5-dihalogéno-2-nitrobenzoate d'alkyle représenté par la formule générale (6) 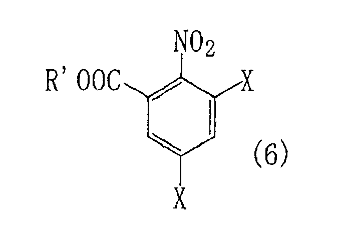 (dans laquelle X et R' sont tels que définis ci-dessus), puis en réduisant le composé de 3,5-dihalogéno-2-nitrobenzoate d'alkyle pour obtenir un composé de 2,4-dihalogéno-6-hydroxyméthylnitrobenzène représenté par la formule générale (7) 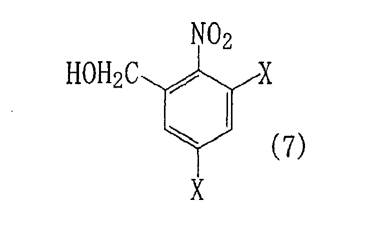 (dans laquelle X est tel que défini ci-dessus), et en soumettant le composé de 2,4-dihalogéno-6-hydroxyméthylnitrobenzène à une alkylation en présence d'une base.

3. Procédé de production d'un dérivé de pyrimidinylacétonitrile présenté dans la revendication 2, dans lequel le composé de 3,5-dihalogéno-2-nitrobenzoate d'alkyle représenté par la formule générale (6) 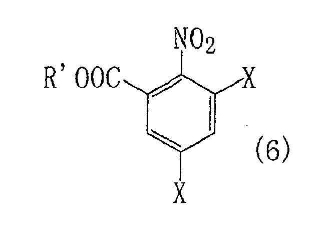 (dans laquelle R' représente un groupe alkyle, et X représente un atome d'halogène) est produit en soumettant un composé d'acide 3,5-dihalogéno-2-nitrobenzoïque représenté par la formule générale (4) 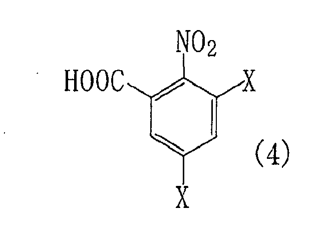 (dans laquelle X est tel que défini ci-dessus) à une alkylation en présence d'une base (méthode A).

4. Procédé de production d'un dérivé de pyrimidinylacétonitrile présenté dans la revendication 2, dans lequel le composé de 3,5-dihalogéno-2-nitrobenzoate d'alkyle représenté par la formule générale (6) 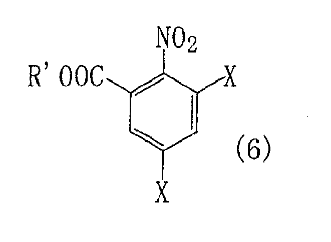 (dans laquelle R' représente un groupe alkyle, et X représente un atome d'halogène) est produit en soumettant un composé de 3,5-dihalogénobenzoate d'alkyle représenté par la formule générale (5) 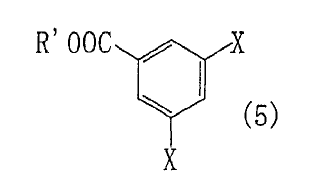 (dans laquelle R' et X sont tels que définis ci-dessus) à une nitration (méthode B).

5. Procédé de production d'un dérivé de pyrimidinylacétonitrile présenté dans la revendication 1, dans lequel le composé de 2,4-dihalogénonitrobenzène représenté par la formule générale (1) 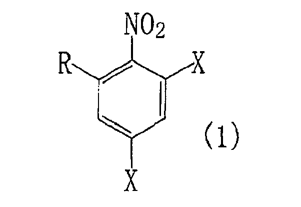 (dans laquelle X représente un atome d'halogène, et R représente un groupe alcoxyméthyle) est produit en réduisant un composé de 3,5-dihalogéno-2-nitrobenzoate d'alkyle représenté par la formule générale (6) 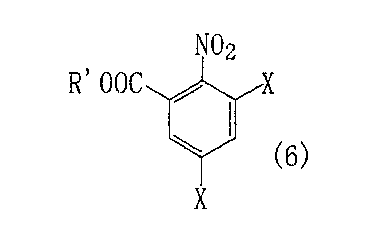 (dans laquelle R' représente un groupe alkyle, et X représente un atome d'halogène) pour obtenir un composé de 2,4-dihalogéno-6-hydroxyméthylnitrobenzène représenté par la formule générale (7) 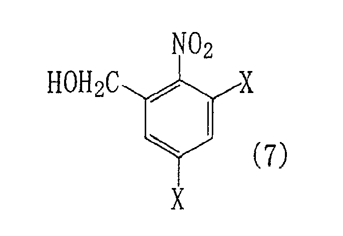 (dans laquelle X est tel que défini ci-dessus), et en soumettant le composé de 2,4-dihalogéno-6-hydroxyméthylnitrobenzène à une alkylation en présence d'une base.

6. Composé de 2,4-dihalogénonitrobenzène représenté par la formule générale (1) 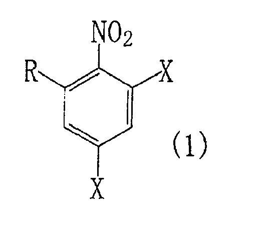 (dans laquelle X représente un atome d'halogène, et R représente un groupe alcoxyméthyle).

7. Composé de 2,4-dihalogéno-6-hydroxyméthylnitrobenzène représenté par la formule générale (7) 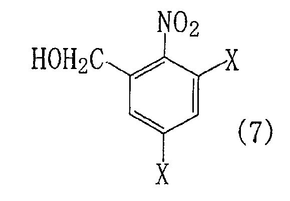 (dans laquelle X représente un atome d'halogène).
